# EUROPEAN PATENT APPLICATION

(11) **EP 2 705 838 A1**
(43) Date of publication of application: **12.03.2014**
(21) Application number: 12183264.6
(22) Date of filing: 06.09.2012
(51) Int. Cl.: A61K 9/14, A61K 9/00, C07D 451/10, A61K 9/16, A61K 31/46

(54) **Tiotropium preparations**

(71) Applicant: XSpray Microparticles AB, 171 65 Solna (SE)
(72) Inventor: Brisander, Magnus, 178 35 EKERÖ (SE); Demirbüker, Mustafa, 175 54 JÄRFÄLLA (SE); Jesson, Gérald, 741 42 KNIVSTA (SE)
(74) Representative: Lundquist, Tomas

(57) **Abstract**

The present invention relates methods of preparing particles of tiotropium bromide particularly useful for inhalation delivery, the particles obtained from such methods, and their use in pharmaceutical compositions for treatment of respiratory disorders, such as COPD (chronic obstructive pulmonary disease) and asthma.

## Description

### Field of the invention

The present invention relates to methods of preparing particles of tiotropium bromide particularly useful for inhalation delivery. Further, the invention relates to the particles obtained from such methods, and their use in pharmaceutical compositions for treatment of respiratory disorders, such as COPD (chronic obstructive pulmonary disease) and asthma.

### Background of the invention

Tiotropium bromide is known from e.g. European patent application EP 418 716 A1 and is an established and effective anticholinergic substance useful in the treatment of respiratory disorders, such as COPD (chronic obstructive pulmonary disease) and asthma. For treating the above-mentioned disorders, the active substance is typically administered by inhalation. In order to provide effective clinical outcome, it is important to provide inhalable powders containing the active substance.

Tiotropium bromide is a substance with high efficacy and typically single doses comprising small amounts of the active substance are required in order to achieve therapeutic effects. In inhalation therapy, the active substance is typically dispersed with excipients and/or carriers in order to provide inhalable preparations where suitable amounts of active substance are delivered to the target organ. The choice of excipient is of importance to provide a powder suitable for inhalation, as the excipient typically provides characteristics, such as size and weight of the powder. Further, the method of producing the powder also represents an important parameter. The average size of the powder particles suitable for inhalation is normally less than 10000 nm with a preference for less than to 3000 nm. The powder particles may be attached to the surface of larger carrier particles, e.g. lactose, and released therefrom before entering the mouth.

Inhalable powders containing tiotropium bromide has previously been described, e.g. in WO 94/28958 and US 7,070,800.

In order to obtain an optimal efficacy with tiotropium bromide, it is desirable that a single inhalation dose of particles containing tiotropium bromide is evenly and widely distributed throughout the lungs down to the alveolar tissue meaning that a large portion of the tiotropium bromide particles form an aerosol that have an optimal alveolar deposition spread enhancing the pharmaceutical efficacy of the active substance. Therefore, one objective of the present invention is to prepare an inhalable powder containing tiotropium bromide, which is released and delivered to the lungs with optimal alveolar deposition spread. A further objective of the present invention is to provide methods of preparing said particles. Yet a further objective of the present invention is to provide improved methods where the characteristics of the particles comprising tiotropium bromide can be altered so that they provide optimal alveolar deposition spread after inhalation.

### Detailed description of the invention

It was surprisingly found that the objectives outlined above can be achieved by means of the particles comprising tiotropium bromide and the methods of preparing them, according to the invention described hereinafter. The methods provide opportunities to obtain improved alternative particles comprising tiotropium bromide that are particularly useful for inhalation delivery.

### Brief description of the drawings

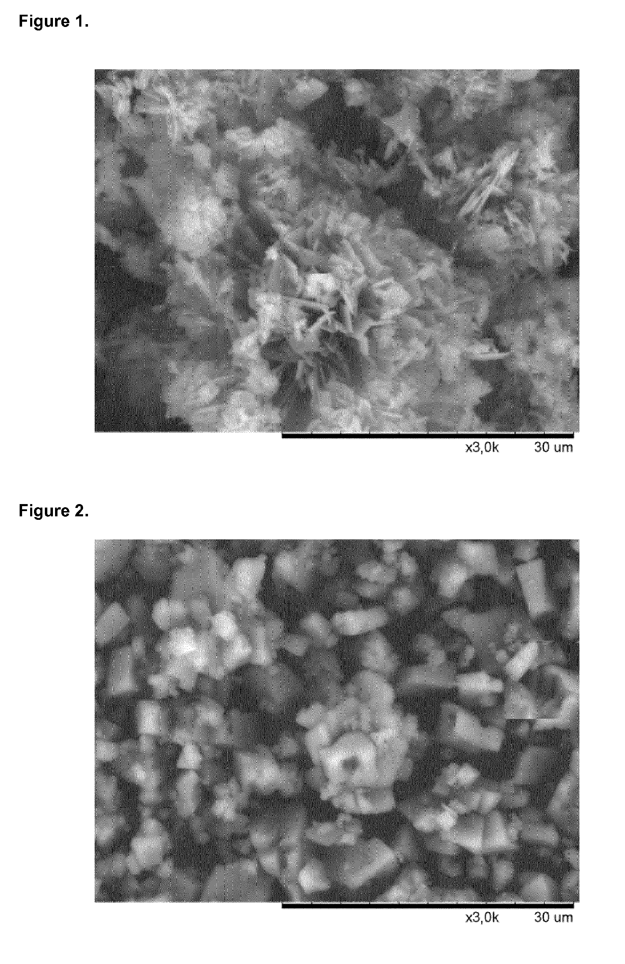

Figure 1 depicts an electron microscope slide with crystalline tiotropium bromide particles, set together into porous sand rose-shaped agglomerates, which provides a free flowing powder of low density. Further details of the experimentation are set out in Example 1.
Figure 2 depicts an electron microscope slide with solid crystalline tiotropium bromide cubic-shaped particles, which provides a free flowing powder of high density. Further details of the experimentation are set out in Example 2.

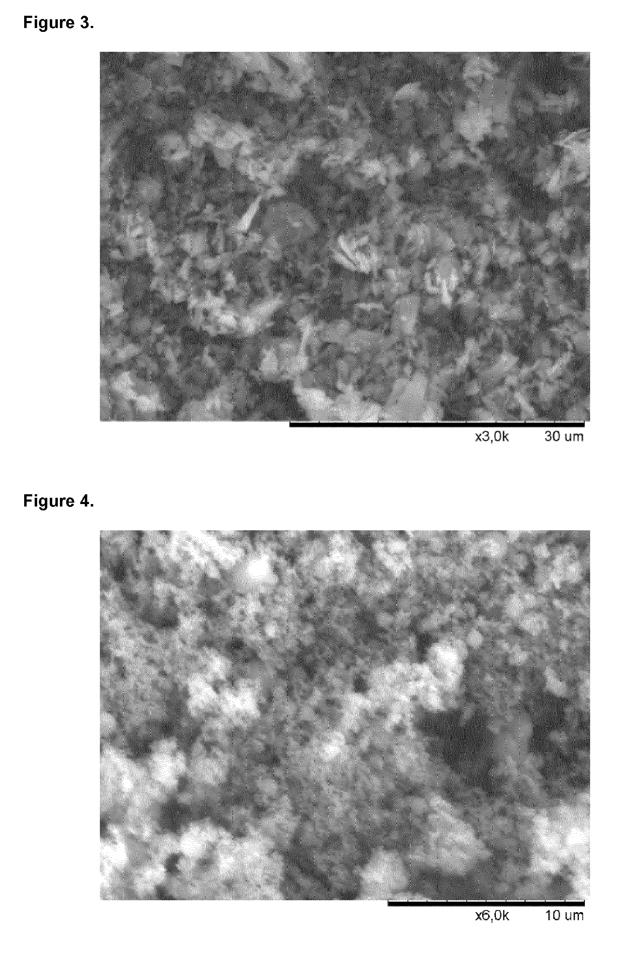

Figure 3 depicts an electron microscope slide with crystalline tiotropium bromide particles produced in supercritical fluid of CO₂, which provides a low or high density powder. Further details of the experimentation are set out in Example 3.
Figure 4 depicts an electron microscope slide with amorphous tiotropium bromide particles, which provides a low density. Further details of the experimentation are set out in Example 4.

By "tiotropium" is meant the compound as a free ammonium cation. The counter-ion (anion) may be chloride, bromide, iodide, methanesulphonate, para-toluenesulphonate or methyl sulphate. Of these anions, the bromide is preferred, i.e "tiotropium bromide".

The inhalable powders according to the invention may for example be administered using inhalers as described elsewhere, e.g. in US 4,570,630A and WO 94/28958.

Methods of the present invention are typically performed in a batch mode or in a continuous mode where the latter in most cases are preferred. Continuous processes in this context means that particle formation is continuously ongoing while at the same time continuously withdrawing/collecting/retaining particles from the mixture after their formation. In particular for precipitation methods, this means that a fluid which is a solution of tiotropium bromide, preferably in the form of a fluid stream, is mixed with an anti-solvent fluid (anti-solvent in relation to tiotropium bromide), preferably in the form of an anti-solvent fluid stream. The mixing of the two fluids is taking place in a mixing function, e.g. a mixing chamber. In the case the process is continuous, i.e. the two fluids are fluid streams, the mixing function typically is associated with a particle formation and separation function permitting the mixed fluid stream to pass through while retaining the particles. Agents modifying the particle characteristics such as size, size distribution and morphology, without being incorporated into the particles may be added to either of the fluids before the mixing step. The fluids typically are conventional liquids or supercritical fluids, where supercritical fluids also includes subcritical fluids (i.e. fluids for which only one of pressure and temperature is above its supercritical value). Typical combinations are a) conventional liquids for both the solution of tiotropium bromide and the antisolvent, b) supercritical solution of tiotropium bromide combined with conventional liquid for the anti-solvent, c) conventional liquid for the solution of tiotropium bromide combined with supercritical fluid for the anti-solvent, and d) supercitical fluids for both of the two fluids. In certain variants the anti-solvent may be omitted. A fluid stream, such as a supercritical containing tiotropium bromide, is then allowed to expand into the particle formation function. These kinds of precipitation methods are discussed in WO 2005061090 (Censdelivery AB), WO 2009072950 (XSpray Microparticles AB), WO 2009072953 (XSpray Microparticles AB), WO 2011159218 (XSpray Microparticles AB) and references cited in these publications. Currently, it is preferred that both of the fluids are conventional liquids in order to obtain crystalline particles of tiotropium bromide in the precipitation methods described in this specification.

The term "anti-solvent" in this specification primarily refers to a fluid which is capable of precipitating tiotropium bromide from a solution containing the tiotropium bromide when mixed with the solution.

The term "solution" encompasses that the solute is either a true solute or minutes particles of colloidal dimensions and less than the particles to be produced.

A preferred particle formation system is the "Right Size system" developed by XSpray microparticles AB, Sweden. A detailed description of the technology can be found in the WO-publications given in the preceding paragraph. An important characteristic of the system is that the two fluid streams should merge within a nozzle at an angle in the interval 45°-135°, with preference for about 90° and sprayed into a particle formation/separation function.

In principle the system allows for producing particles of predetermined size and/or morphology. Here the Right Size system and apparatus will be described using the non-limiting example of using tiotropium bromide in the form of a liquid solution and CO₂ as a supercritical fluid anti-solvent for tiotropium bromide. This is also illustrated with conventional liquids as solution and antisolvents, respectively, further below in the specification.

This basic system consists of one pumping set-up for the tiotropium bromide dissolved in a liquid solvent and one pumping set-up for an anti-solvent, for example CO₂, however other anti-solvents may be used when suitable. Each pumping set-up includes instruments such as a flow meter and a pressure meter that are used to control the process conditions. These two pumping set-ups are fluidically connected at a spray nozzle.

A stream of tiotropium bromide solution is mixed with a stream of CO₂ under flow conditions within the spray nozzle. The mixed stream is then sprayed at the outlet of the nozzle into a precipitation vessel under controlled conditions (typically pressure and temperature). CO₂ acts as an anti-solvent. Tiotropium bromide precipitates to form an essentially amorphous phase and/or an essentially crystalline phase, which during the process is formed as fine particles. Particles are retained in the vessel by a filtering set-up. A back pressure regulator is typically used to control the pressure inside the precipitation vessel.

When using the system, the system operator typically starts with equilibrating the system by pumping CO₂, a "tiotropium bromide-like solution" (a solution similar in composition to the tiotropium bromide solution but containing no tiotropium bromide) through the system until flow rates, pressure and temperature have reached a desired steady state. Critical parameters for setting up the system are tiotropium bromide solution composition, tiotropium bromide solution flow rate, CO₂ flow rate, CO₂ pressure and temperature, nature and amount/concentration of the optional modifier and modifier flow rate, if such is used.

Next, the "tiotropium bromide-like solution" is exchanged for the tiotropium bromide solution and particles are produced and retained downstream of the mixing, e.g. downstream of the outlet of the nozzle. Afterwards, the system is typically cleaned by pumping the "tiotropium bromide-like solution" through the system. Subsequently the solvent pumps for the solution and the modifier, if used, are stopped and the powder is dried by flushing CO₂ through the retained particles in order to extract any remaining solvent. The precipitation vessel is then depressurized and the particles can be collected.

In one aspect of the invention, there is provided a method of preparing particles of tiotropium bromide with an average particle size of from about 100 nm to 10000 nm, comprising
a) dissolving tiotropium bromide in an organic solvent to obtain a liquid solution;
b) providing a stream of the liquid solution, preferably pressurized, which is mixed with a stream of an antisolvent, preferably pressurized; and
c) wherein the mixed stream is sprayed at the outlet of the nozzle into a precipiation vessel.

In one embodiment of this aspect, said particles of tiotropium bromide have an average particle size of from about 100 nm to 3000 nm.

In another embodiment of this aspect, said particles are crystalline particles of tiotropium bromide.

In another embodiment of this aspect, said particles are amorphous particles of tiotropium bromide.

In another embodiment of this aspect, the mixed stream sprayed into the vessel is a suspension.

In another embodiment of this aspect, said suspension is agitated agitated for a predetermined period of time before it is filtered filtered and dried to obtain a free flowing powder.

In another embodiment of this aspect, the free flowing powder comprises a low density particle powder of tiotropium bromide.

In another embodiment of this aspect, the organic solvent is dimethyl sulfoxide, N-methyl pyrrolidone, methanol, water, acetone, acetonitrile, isopropanol, PEG 400 or 2,2,2-trifluoroethanol, or a mixture of any of these.

In another embodiment of this aspect, the organic solvent is 2,2,2-trifluoroethanol.

In another embodiment of this aspect, the antisolvent is supercritical CO₂, methyl tert-butyl ether, ethyl acetate, tetrahydrofurane or n-hexane or a mixture thereof.

In another embodiment of this aspect, the antisolvent is supercritical CO₂, methyl tert-butyl ether, ethyl acetate, or a mixture of methyl tert-butyl ether and ethyl acetate.

In another embodiment of this aspect, the particles of tiotropium bromide have an average particle size of from about 100 nm to 1000 nm. Preferably, then, the organic solvent is DMSO and the antisolvent is supercritical CO₂.

In another embodiment of this aspect, the particles are amorphous nanoparticles of tiotropium bromide, preferably providing a powder of low density.

In another aspect of the invention, there is provided an inhalable powder comprising tiotropium bromide, wherein said tiotropium bromide is in the form of particles, obtainable with methods as set out above.

In another aspect of the invention, there is provided an inhalable tiotropium bromide composition comprising particles obtainable with methods as set out above, in admixture with a physiologically acceptable excipient, wherein said particles are distributed throughout the lungs down to the alveolar tissue in order to obtain an optimal alveolar deposition spread.

In one embodiment of this aspect, glucose or lactose or mixtures thereof are used as excipients.

Optimal alveolar deposition spread of an inhaled powder may be measured with various available techniques, e.g. as described in WO 09/002267, or by lung scintigraphy. These techniques allow to measure how large is the fraction of powder that form a aerosol and/or what is the size distribution of the particles forming this fraction and/or how this fraction is distributed through the lung down to the alveolar tissue and/or how large is the enhancement of the pharmaceutical efficacy at the alveolar tissue.

The degree of density of a powder and the degree of porosity of the particles providing the powder, i.e. "low density powder" or "powder of low density " may be measured according to general methods and typically, the mass of the powder is measured with a balance, and its volume measured. Bulk and tap density is preferably measured as described in The United States Pharmacopeia (*No. 616 Bulk density and Tapped density of powders, Stage 6, Harmonization 2011*)*.* Measurement of density of particles as such may be measured by various methods available to the skilled person.

The degree of flowability may be defined as a percentage of compressibility (also denoted Carr's index). This represents the tapped density minus bulk density)/ tapped density x 100. The term "free flowing" is here defined as % compressibility below 28% or more preferably below 15%.

The methods of the invention provide the possibility to obtain an inhalable powder of tiotropium bromide of low density with crystalline or amorphous particles of tiotropium bromide, and which also is free flowing.

In another aspect of the invention, there is provided an inhalable powder composition as set out above, for use in treating a disease that is responsive to the administration of tiotropium bromide.

In one embodiment of this aspect, said disease is asthma or COPD.

In another aspect of the invention, there is provided a method of treating a disease that is responsive to the administration of tiotropium bromide, comprising administering to a host in need thereof an inhalable powder composition, as set out above.

In one embodiment of this aspect, said disease is asthma or COPD.

In another aspect of the invention, there is provided an inhalable powder comprising tiotropium bromide particles, wherein said particles are porous, crystalline tiotropium bromide particles providing a free flowing powder of low density.

In another aspect of the invention, there is provided an inhalable powder comprising tiotropium bromide particles, wherein said particles are solid crystalline tiotropium bromide particles providing free flowing powder of high density.

In another aspect of the invention, there is provided an inhalable powder comprising tiotropium bromide particles, wherein said particles are amorphous tiotropium bromide particles with an average particle size of from about 100 nm to 1000 nm.

The inhalable powders according to the invention are **characterized in that** the tiotropium bromide powder has an average particle size of from about 100 to 10000 nm, preferably from about 100 to 3000 nm. The phrase average particle size used here denotes the 50% value from the volume distribution measured with a laser diffractometer using the dry dispersion method.

The inhalable powders comprising tiotropium bromide according to the invention are characterised, in accordance with the objective on which the present invention is based, by characteristics that provide optimal distribution throughout the lungs down to the alveolar tissue in order to obtain an optimal alveolar deposition spread, such as crystalline or amorphous particles providing a powder of low density. The parameters in methods of preparing the inhalable tiotropium bromide powders may be altered so that the particles are varied, and thereby the methods are particularly suitable in the identification of inhalable powders comprising tiotropium bromide with improved properties.

If the inhalable powder is to be administered in suitable inhalers, the preparation of the inhalable powders is followed by the manufacture of the powder-filled capsules.

Within the scope of the present invention, tiotropium bromide is preferred of all the tiotropium salts. References to tiotropium bromide within the scope of the present invention should always be taken as references to all possible amorphous and crystalline modifications of tiotropium bromide. These may, for example, include molecules of solvent in their crystalline structure. Of all the crystalline modifications of tiotropium bromide, those which also include water (hydrates) are preferred according to the invention. It is particularly preferable to use tiotropium bromide monohydrate within the scope of the present invention.

### Preparation of crystalline tiotropium bromide

Crystalline tiotropium bromide in its anhydrate or monohydrate form was prepared using a Xspray's Right Size system adapted for liquid antisolvent. Here the apparatus will be described using the non-limiting example of using tiotropium bromide in the form of a liquid solution and an organic solvent used as an antisolvent fluid for tiotropium bromide. The solvent used for diluting the substance drug may then be present in either one or both of the tiotropium solution and the antisolvent fluid depending on the solubility characteristics of this substance drug.

This basic system consists of one pumping set-up for the tiotropium bromide dissolved in a liquid solvent or mixture of liquid solvents and one pumping set-up for the anti-solvent, for example a mixture of liquid solvents. Each pumping set-up may include instruments such as a flow meter and a pressure meter that are used to control the process conditions. These two pumping set-ups are fluidically connected at a spray nozzle or mixing device that permit a continuous and efficient mixing reducing concentration gradient in the resulting fluid mixture and reducing the size distribution of the precipitating material.

A stream of tiotropium bromide solution is mixed with a stream of antisolvent under flow conditions within the spray nozzle. The mixed stream is then sprayed at the outlet of the nozzle into a precipitation vessel. The temperature of the fluids to be mixed is controlled by using heat exchangers and the precipitation vessel can be thermostated. In one embodiment of the set-up the pressure at which the precipitation occurs can be controlled by adapting a back pressure regulator at the outlet of a closed precipitation vessel. Tiotropium bromide precipitates in form of a suspension that is collected in the precipitation vessel. A mixing device can be placed in the precipitation vessel in order to maintain the suspension agitated. This agitation results in a shear force applied to the crystalline particles and reduce sedimentation and agglomeration of particles while the crystallization process is on-going.

When using the system, the system operator typically starts with equilibrating the system by pumping the antisolvent and a priming solution (consisting of the solvent or mixture of solvents used to dissolve tiotropium bromide) through the system until flow rates, pressure and temperature have reached a desired steady state. Critical parameters for setting up the system are tiotropium bromide solution composition, tiotropium bromide solution flow rate, tiotropium bromide solution temperature, antisolvent composition, antisolvent flow rate, antisolvent temperature and temperature, pressure and agitation speed in the precipitation vessel.

Next, the priming solution is exchanged for the tiotropium bromide solution and a particle suspension is produced and collected downstream of the mixing, e.g. downstream of the outlet of the nozzle. Afterwards, the system is typically cleaned by pumping the priming solution. Then pumps are stopped but agitation that can be maintained for a defined period for particle maturation. The suspension is then filtered and the resulting filtrate is dried using standard pharmaceutical techniques. The result is a free flowing powder that can be used for inhalation. Precipitation can be made in a high pressure vessel and sub- and/or supercritical CO₂ can be used for solvent removing and drying of particles.

### Example 1. Production of porous crystalline tiotropium bromide particles providing a free flowing powder of low density

A 10% (w /v) tiotropium bromide anhydrate solution was prepared in pure 2,2,2-trifluoroethanol (TFE). The solution was filtered through a 0.45 µm polytetrafluoroethylene (PTFE) membrane filter before used. A parallel mixture of methyl tert-butyl ether and ethyl acetate (1:1 v/v) was prepared as antisolvent.

The system was thermostated to 25°C and the flow rate for tiotropium bromide and antisolvent were set respectively to 5 and 25 ml/min. The suspension was collected in a 100 ml precipitation vessel and the magnetic agitator was rotated at 250 rpm.

The suspension was subsequently filtered through a 2 µm sintered metal filter. The filtrate was collected and vacuum dried. The result was a low density powder of free flowing crystalline particles of tiotropium bromide, set together into porous sand rose-shaped agglomerates.

### Example 2. Production of solid crystalline tiotropium bromide particles providing a free flowing powder of high density

An 8.3% (w /v) tiotropium bromide anhydrate solution was prepared in a mixture of 2,2,2-trifluoroethanol (TFE) and water (5:1). The solution was filtered through a 0.45 µm polytetrafluoroethylene (PTFE) membrane filter before used. Pure methyl tert-butyl ether was used as antisolvent.

The system was thermostated to 25°C and the flow rate for tiotropium bromide and antisolvent were set respectively to 5 and 25 ml/min. The suspension was collected in a 100 ml precipitation vessel and the magnetic agitator was rotated at 750 rpm.

The suspension was subsequently filtered through a 2 µm sintered metal filter. The filtrate was collected and vacuum dried. The result was a free flowing high density powder made of solid crystalline cubic-shaped particles of tiotropium bromide.

### Example 3. Production of crystalline particles of tiotropium bromide in supercritical fluid of CO₂

A 10% (w /v) tiotropium bromide anhydrate solution was prepared in pure 2,2,2-trifluoroethanol (TFE). The solution was filtered through a 0.45 µm polytetrafluoroethylene (PTFE) membrane filter before to be used. In parallel mixture of methyl tert-butyl ether and ethyl acetate (1:1 v/v) was prepared as antisolvent.

The high pressure vessel was filled with CO₂ at 25°C and 55 bars. The flow rate for tiotropium bromide and antisolvent were set respectively to 2 and 10 ml/min. The suspension was collected the high pressure vessel without agitation.

At the end of the precipitation process, the suspension was then dried by pumping supercritical CO₂ at 40°C and 120 bars and a flow rate of 50 g/min. The result was a powder of crystalline particles of tiotropium bromide.

### Experimental description for the preparation of particles of amorphous tiotropium bromide

The preparation of crystalline tiotropium bromide in its anhydrate or monohydrate form were prepared using a Xspray's Right Size system adapted for the use of sub- and/or super-critical fluid as antisolvent. Here the apparatus will be described using the non-limiting example of using tiotropium bromide in the form of a liquid solution and CO₂ as a supercritical anti-solvent fluid for tiotropium bromide. The solvent used for diluting the substance drug may then be present in either one or both of the tiotropium bromide solution and the antisolvent fluid depending on the solubility characteristics of this substance drug.

This basic system consists of one pumping set-up for the tiotropium bromide dissolved in a liquid solvent and one pumping set-up for an anti-solvent, for example CO₂, however other anti-solvents may be used when suitable. Each pumping set-up includes instruments such as a flow meter and a pressure meter that are used to control the process conditions. These two pumping set-ups are fluidically connected at a spray nozzle.

A stream of tiotropium bromide solution was mixed with a stream of CO₂ under flow conditions within the spray nozzle. The mixed stream was then sprayed at the outlet of the nozzle into a precipitation vessel under controlled conditions (typically pressure and temperature). CO₂ acts as an anti-solvent. Tiotropium bromide precipitated into fine amorphous particles. Particles were retained in the vessel by a filtering set-up. A back pressure regulator was typically used to control the pressure inside the precipitation vessel.

For preparing tiotropium bromide particles, it may be advantageous to have an extra pumping set-up for injecting an additional solvent, referred to as a modifier, into the CO₂. Here a pumping set-up control was set up for the modifier and the modifier is mixed with the CO₂ in a mixer before entering the nozzle.

When using the system, the system operator typically starts with equilibrating the system by pumping CO₂, a priming solution (consisting of the solvent or mixture of solvents used to dissolve tiotropium bromide) and the modifier (if used) through the system until flow rates, pressure and temperature have reached a desired steady state. Critical parameters for setting up the system are tiotropium bromide solution composition, tiotropium bromide solution flow rate, CO₂ flow rate, CO₂ pressure and temperature, nature and amount/concentration of the optional modifier and modifier flow rate, if such is used.

Next, the priming solution is exchanged for the tiotropium bromide solution and particles are produced and retained downstream of the mixing, e.g. downstream of the outlet of the nozzle. Afterwards, the system is typically cleaned by pumping the priming solution through the system. The particles are dried by flushing CO₂ through the retained particles in order to extract any remaining solvent. The precipitation vessel is then depressurized and the particles can be collected.

### Example 4. Production of amorphous particles of tiotropium bromide providing a powder of low density

A 5% (w /v) tiotropium bromide anhydrate solution was prepared in pure dimethyl sulfoxide (DMSO). The solution was filtered through a 0.45 µm polytetrafluoroethylene (PTFE) membrane filter before to be used.

The high pressure vessel was filled with CO₂ at 40°C and 120 bars. The flow rate for tiotropium bromide and CO₂ (antisolvent) were set respectively to 1 ml/min and 100 g/min. Drying was carried out for 3 minutes with a CO₂ flow rate of 100 g/min.

The resulting powder was a low density powder of amorphous nanoparticles of tiotropium bromide.

Non-limiting, itemized list of aspects and embodiments of the invention:
A. A method of preparing particles of tiotropium bromide with an average particle size of from about 100 nm to 10000 nm, comprising
   a) dissolving tiotropium bromide in an organic solvent to obtain a liquid solution;
   b) providing a stream of the liquid solution, preferably pressurized, which is mixed with a stream of an antisolvent, preferably pressurized; and
   c) wherein the mixed stream is sprayed at the outlet of a nozzle into a precipitation vessel.
B. The method of item A, wherein said particles of tiotropium bromide have an average particle size of from about 100 nm to 3000 nm.
C. The method of item A or B, wherein said particles are crystalline particles of tiotropium bromide.
D. The method of item A or B, wherein said particles are amorphous particles of tiotropium bromide.
E. The method according to any one of items A to D, wherein the mixed stream sprayed into the vessel is a suspension.
F. The method of item E, wherein the suspension is agitated for a predetermined period of time before it is filtered and dried to obtain a free flowing powder.
G. The method of item F, wherein said free flowing powder comprises a low density particle powder of tiotropium bromide.
H. The method according to any one of items A to G, wherein said organic solvent is 2,2,2-trifluoroethanol.
I. The method according to any one of items A to H, wherein said antisolvent is supercritical CO₂, or methyl tert-butyl ether, ethyl acetate or a mixture of methyl tert-butyl ether and ethyl acetate.
J. The method according to item A, wherein said particles of tiotropium bromide has an average particle size of from about 100 nm to 1000 nm.
K. The method of item J, wherein the organic solvent is DMSO and the antisolvent is supercritical CO₂.
L. The method of item J or K, wherein said particles are amorphous nanoparticles of tiotropium bromide, preferably a powder of low density.
M. An inhalable powder comprising tiotropium bromide, wherein said tiotropium bromide is in the form of particles, obtainable with methods according to any one of items A to L.
N. An inhalable tiotropium bromide composition comprising particles obtainable with methods according to any one of items A to L, in admixture with a physiologically acceptable excipient, wherein said particles are distributed throughout the lungs down to the alveolar tissue in order to obtain an optimal alveolar deposition spread.
O. The composition according to item N, wherein glucose or lactose or mixtures thereof are used as excipients.
P. An inhalable powder composition according to item N or O, for use in treating a disease that is responsive to the administration of tiotropium bromide.
Q. The composition for use, according to item P, wherein said disease is asthma or COPD.
R. A method of treating a disease that is responsive to the administration of tiotropium bromide, comprising administering to a host in need thereof an inhalable powder composition, according to item N or O.
S. The method of item R, wherein said disease is asthma or COPD.
T. An inhalable powder comprising tiotropium bromide particles, wherein said particles are porous, crystalline tiotropium bromide particles providing a free flowing powder of low density.
U. An inhalable powder comprising tiotropium bromide particles, wherein said particles said particles are solid crystalline tiotropium bromide particles providing free flowing powder of high density.
V. An inhalable powder comprising tiotropium bromide particles, wherein said particles are amorphous tiotropium bromide particles with an average particle size of from about 100 nm to 1000 nm.

## Claims

1. A method of preparing particles of tiotropium bromide with an average particle size of from about 100 nm to 10000 nm, comprising
a) dissolving tiotropium bromide in an organic solvent to obtain a liquid solution;
b) providing a stream of the liquid solution, preferably pressurized, which is mixed with a stream of an antisolvent, preferably pressurized; and
c) wherein the mixed stream is sprayed at the outlet of a nozzle into a precipitation vessel.

2. The method of claim 1, wherein said particles of tiotropium bromide have an average particle size of from about 100 nm to 3000 nm.

3. The method of claim 1 or 2, wherein said particles are crystalline particles of tiotropium bromide.

4. The method according to any one of claims 1 to 3, wherein the mixed stream sprayed into the vessel is a suspension.

5. The method of claim 4, wherein the suspension is agitated for a predetermined period of time before it is filtered and dried to obtain a free flowing powder.

6. The method according to any one of claims 1 to 5, wherein said organic solvent is 2,2,2-trifluoroethanol.

7. The method according to any one of claims 1 to 6, wherein said antisolvent is supercritical CO₂, or methyl tert-butyl ether, ethyl acetate or a mixture of methyl tert-butyl ether and ethyl acetate.

8. The method according to claim 1, wherein said particles of tiotropium bromide has an average particle size of from about 100 nm to 1000 nm.

9. The method of claim 8, wherein the organic solvent is DMSO and the antisolvent is supercritical CO₂.

10. The method of claim 8 or 9, wherein said particles are amorphous nanoparticles of tiotropium bromide, preferably a powder of low density.

11. An inhalable powder comprising tiotropium bromide, wherein said tiotropium bromide is in the form of particles, obtainable with methods according to any one of claims 1 to 10.

12. An inhalable tiotropium bromide composition comprising particles obtainable with methods according to any one of claims 1 to 10, in admixture with a physiologically acceptable excipient, wherein said particles are distributed throughout the lungs down to the alveolar tissue in order to obtain an optimal alveolar deposition spread.

13. An inhalable powder composition according to claim 12, for use in treating a disease that is responsive to the administration of tiotropium bromide.

14. An inhalable powder comprising tiotropium bromide particles, wherein said particles are porous, crystalline tiotropium bromide particles providing a free flowing powder of low density.

15. An inhalable powder comprising tiotropium bromide particles, wherein said particles are amorphous tiotropium bromide particles with an average particle size of from about 100 nm to 1000 nm.
